# EUROPÄISCHE PATENTSCHRIFT

(11) **EP 2 934 615 B1**
(45) Veröffentlichungstag und Bekanntmachung des Hinweises auf die Patenterteilung: **24.10.2018**
(21) Anmeldenummer: 13811344.4
(22) Anmeldetag: 12.12.2013
(51) Int. Cl.: A61M 1/00, A61M 1/06

(54) **BRUSTHAUBENEINHEIT MIT MEDIENTRENNUNG**
BREAST SHIELD UNIT HAVING MEDIA SEPARATION
UNITÉ TÉTERELLE POURVUE D'UNE SÉPARATION DE MILIEUX

(30) Priorität: 18.12.2012 CH 28392012
(43) Veröffentlichungstag der Anmeldung: 28.10.2015
(73) Patentinhaber: Medela Holding AG, 6340 Baar (CH)
(72) Erfinder: FURRER, Etienne, CH-6332 Hagendorn (CH); RIGERT, Mario, CH-6033 Buchrain (CH); SCHLIENGER, André, CH-8933 Maschwanden (CH)
(74) Vertreter: Clerc, Natalia
(86) Internationale Anmeldenummer: PCT/CH2013/000220
(87) Internationale Veröffentlichungsnummer: WO 2014/094187

(56) Entgegenhaltungen:
- EP-A1- 1 034 807
- EP-A1- 2 308 523
- EP-A1- 2 441 481
- US-A1- 2004 087 898
- US-A1- 2007 060 873

## Beschreibung

### TECHNISCHES GEBIET

Die vorliegende Erfindung betrifft eine Brusthaubeneinheit zur Verwendung mit einer Brustpumpe zum Abpumpen von menschlicher Muttermilch.

### STAND DER TECHNIK

Brustpumpen zum Abpumpen von menschlicher Muttermilch mittels Unterdruck sind hinlänglich bekannt. Auf dem Markt sind manuell betriebene wie auch motorisch betriebene Brustpumpen erhältlich. Sie sind entweder direkt oder über eine Vakuumleitung mit einer Brusthaube verbunden. Die Brusthaube wird an die abzupumpende Mutterbrust angelegt, so dass mindestens die Brustwarze, meistens auch die Areola und das umliegende Gewebe der Mutterbrust, dichtend umschlossen sind. Die Brusthaube ist üblicherweise entweder direkt oder über eine Milchleitung mit einem Milchsammelbehälter verbunden, so dass abgepumpte Milch direkt in diesen Behälter fliessen kann.

Ferner sind Medientrenneinrichtungen für Brustpumpen bekannt. Sie dienen dazu, die Vakuumpumpe vor Verschmutzung durch Milch zu schützen.

US 2004/0087898 offenbart eine Brusthaubeneinheit mit einem Kopplungsteil zur Verbindung mit einer Brustpumpe. In diesem Anschlussteil ist eine flexible Medientrennmembran angeordnet, welche das angelegte Vakuum in den Hohlraum des Brusthaubentrichters überträgt und gleichzeitig einen Milchflussweg in der Brusthaube von einer Vakuumanschlussöffnung trennt.

WO 2008/057218 und WO 2011/0387841 zeigen ebenfalls derartige Medientrennmembranen, welche in einem Kopplungsteil angeordnet sind.

EP 2 308 523 A1 offenbart eine Brusthaubeneinheit mit einer Medientrennmembran, welche die Brustwarze kontaktieren und somit massieren kann.

US 2007/0060873 A1 beschreibt eine Brustpumpe mit einer Medientrennmembran, welche beabstandet vom Stutzen der Brusthaube angeordnet ist.

Obwohl diese Medientrennvorrichtungen bezüglich Schutz der Saugleitung und der Brustpumpe Vorteile bringen, vergrössern sie das Volumen der Brusthaube doch massiv. Dies ist insbesondere bei sogenannten "Hands-free"-Lösungen, bei welchen die Brusthaube unter der Kleidung in einem Büstenhalter getragen wird, ungeeignet.

EP 1 034 807 A1 zeigt eine Brusthaube mit einem Liner, welcher von aussen mit Druck beaufschlagbar ist, um die Brustwarze zu massieren.

In EP 2 441 481 A1 ist eine Pumpmembran beabstandet zum Stutzen der Brusthaube angeordnet.

### DARSTELLUNG DER ERFINDUNG

Es ist deshalb eine Aufgabe der Erfindung, eine Brusthaubeneinheit mit einer Medientrenneinrichtung zu schaffen, welche möglichst kompakt und klein ausgebildet ist.

Diese Aufgabe lösen eine Brusthaubeneinheit mit den Merkmalen des Patentanspruchs 1.

Die erfindungsgemässe Brusthaubeneinheit zur Verwendung mit einer Brustpumpe zum Abpumpen von menschlicher Muttermilch weist eine Brusthaube zur Aufnahme einer Mutterbrust und eine Medientrenneinrichtung zur Übertragung eines Vakuums in das Innere der Brusthaube auf. Die Brusthaubeneinheit weist einen Aufnahmebereich zur Aufnahme einer Brustwarze der Mutterbrust auf. Die Medientrenneinrichtung weist eine Medientrennmembran auf, welche zwecks Übertragung des Vakuums zwischen zwei Positionen hin und her bewegbar ist. Erfindungsgemäss befindet sich die Medientrennmembran mindestens in einer der zwei genannten Positionen im Aufnahmebereich bzw. sie grenzt, wenn sie unterhalb des Aufnahmebereichs angeordnet ist, an diesen an.

Da die Medientrennmembran im Bereich der Brustwarze angeordnet ist, kann die Brusthaubeneinheit trotz Medientrenneinrichtung äusserst kompakt und klein ausgebildet werden. Ein weiterer wesentlicher Vorteil ist, dass das Totvolumen, d.h. das beim Abpumpen zu evakuierende Volumen in der Brusthaube weiter minimiert ist.

Der Aufnahmebereich der Brustwarze und der für die Übertragung des Vakuums notwendige Bewegungsraum der Medientrennmembran teilen sich teilweise denselben physischen Raum. Trotzdem stören sich Brustwarze und Membran nicht gegenseitig, da die Bewegungen synchron erfolgen. D.h. wenn sich die Membran von der Brust weg bewegt, bewegt sich die Brustwarze, aufgrund des im Aufnahmebereich übertragenen Vakuums zur Membran hin und hat, da sich die Membran aus dem Aufnahmebereich genügend entfernt hat, auch genügend Platz sich auszudehnen. Dehnt sich die Membran anschliessend aufgrund der zyklischen Pumpbewegung der Vakuumpumpe wieder in den Aufnahmebereich hinein aus, so zieht sich die Brustwarze aufgrund des Anstiegs des Drucks im Aufnahmebereich auf ihre annähernd natürliche Länge hin zurück. Deshalb wird die Membran in ihrer Bewegung nicht gestört und sie kann sich maximal in den Aufnahmebereich hin ausdehnen. Es besteht somit keinerlei Gefahr, dass die Medientrennmembran in ihrer Wirkungsweise beeinträchtigt wird. Auch das Risiko, dass die Medientrennmembran die Brustwarze berühren bzw. an ihr anhaften könnte und somit den Milchfluss unterdrücken könnte, ist praktisch inexistent.

In einem bevorzugten Ausführungsbeispiel ist die Medientrennmembran in der Verlängerung der Mutterbrust an der Hinterseite der Brusthaubeneinrichtung angeordnet. Derartige Brusthaubeneinheiten lassen sich einfach und kostengünstig herstellen.

Es ist ein rohrförmiger Stutzen vorhanden, welcher den Aufnahmebereich bildet und welcher ein Ende aufweist, wobei die Medientrennmembran unmittelbar an diesem Ende angeordnet ist. Der Stutzen ist vorzugsweise ein Teil der Brusthaube oder eines Brusthaubeneinsatzes.

In einer bevorzugten Ausführungsform weist die Brusthaubeneinheit einen Anschluss zur Verbindung mit einem Milchsammelbehälter auf, wobei die Medientrennmembran annähernd senkrecht zu diesem Anschluss angeordnet ist. Unter "annähernd senkrecht" wird hier auch ein Winkel verstanden, welcher sich von 0° bis 20°, vorzugsweise von 0° bis 5° vom rechten Winkel unterscheidet.

In einer anderen bevorzugten Ausführungsform ist ein Milchflussweg vorhanden, welcher vom Aufnahmebereich der Brusthaube zu einem Anschluss zur Verbindung mit einem Milchsammelbehälter führt, wobei die Medientrenneinrichtung in diesem Milchflussweg angeordnet ist. Vorzugsweise unterbricht die Medientrennmembran den Milchflussweg nach Massgabe eines zyklisch angelegten Unterdrucks und gibt ihn wieder frei. Dies vermindert das Totvolumen.

In einer bevorzugten Ausführungsform weist die Brusthaubeneinheit einen Anschluss zur Verbindung mit einem Milchsammelbehälter auf, wobei die Medientrennmembran annähernd waagrecht zu diesem Anschluss angeordnet ist. Unter "annähernd waagrecht" wird hier auch ein Neigungswinkel verstanden, welcher 0° bis 20°, vorzugsweise 0° bis 5° beträgt.

Die drei oben stehenden Varianten haben den Vorteil, dass die Brusthaubeneinheit in Längsrichtung der Brusthaube äusserst kurz und kompakt gestaltet werden kann.

In einer bevorzugten Ausführungsform ist ein Rückschlagventil vorhanden, welches den Aufnahmebereich von einem Anschluss zur Verbindung mit einem Milchsammelbehälter trennt, wobei das Rückschlagventil einstückig mit der Medientrennmembran ausgebildet ist. Dies reduziert die Herstellungskosten.

Die Medientrenneinrichtung ist als Ganzes relativ zur Brusthaube in Längsrichtung bewegbar angeordnet.

Vorzugsweise weist die Brusthaubeneinheit ferner einen Brusthaubeneinsatz auf, welcher in der Brusthaube befestigbar ist, wobei die Medientrenneinrichtung mittels dieses Brusthaubeneinsatzes in der Brusthaube bewegbar aufgehängt ist.

In einer bevorzugten Ausführungsform weist die Brusthaube sich in einer Längsrichtung der Brusthaube erstreckende Führungsschlitze auf, entlang welcher die Medientrenneinrichtung als Ganzes bewegbar ist. Dadurch ist die Bewegung geführt und begrenzt.

Die Medientrenneinrichtung weist ein formstabiles Gehäuse auf, wobei die Medientrennmembran darin bewegbar gehalten ist.

In einer bevorzugten Ausführungsform weist die Brusthaube an einem der Mutterbrust abgewandten Ende eine rückseitige Öffnung auf, wodurch die Medientrenneinrichtung durch Zugang über diese rückseitige Öffnung in Längsrichtung zur Mutterbrust hin manuell verschiebbar, insbesondere drückbar ist. Dies erleichtert das Aufsetzen und Anpassen der Brusthaubeneinheit an die Mutterbrust, insbesondere wenn eine Brusthaube bzw. ein Brusthaubeneinsatz mit einer flexiblen Brustauflage verwendet wird. Vorzugsweise ist die Medientrennvorrichtung mittels der flexiblen Brustauflage am Gehäuse aufgehängt. In einer bevorzugten Ausführungsform erfolgt diese Aufhängung dadurch, dass ein umlaufender Befestigungskragen der Brustauflage über einen Flansch der Brusthaube gestülpt ist. Die Brustauflage lässt sich nach vorne aus der Brusthaube heraus drücken. Je nach Form lässt sie sich sogar umstülpen. Wird nun diese Brustauflage an die Brust angelegt, so nimmt sie automatisch die Form der Brust an. Je nach Form klappt sie dabei in diese der Brust angepasste Form um.

In einer bevorzugten Ausführungsform ist die Brusthaube formstabil ausgebildet. Die Brusthaubeneinheit weist ferner einen flexiblen Brusthaubeneinsatz zur Einlage in die Brusthaube auf, wobei die Brusthaube ein erstes offenes Brusthaubenende zur Anlage an eine Mutterbrust aufweist und wobei der Brusthaubeneinsatz ein erstes und ein zweites Einsatzende aufweist, welche eine Längsrichtung definieren. Das erste offene Brusthaubenende weist einen umlaufenden Rand auf, an welchem der Brusthaubeneinsatz mit dem ersten Einsatzende befestigbar oder befestigt ist, wobei sich der Brusthaubeneinsatz im montierten Zustand von diesem ersten Einsatzende im Innern der Brusthaube zum zweiten Einsatzende erstreckt. Der Brusthaubeneinsatz verläuft zwischen dem ersten und dem zweiten Einsatzende im Wesentlichen beabstandet zur Brusthaube, wobei das zweite Einsatzende im montierten Zustand des Brusthaubeneinsatzes in Längsrichtung relativ zur Brusthaube verschiebbar ist. Vorzugsweise ist die Medientrenneinrichtung mit dem zweiten Einsatzende verbunden.

Diese Ausführungsform verhindert, dass die Mutter einen zu starken und/oder einen ungleichmässig verteilten Druck auf die Brusthaube und somit auf die Brust ausübt. Dies wäre auf die Dauer schmerzhaft und verhindert einen optimalen Milchfluss aus der Brust.

Die erfindungsgemässe Medientrenneinrichtung weist in einer Ausführungsform ein formstabiles Gehäuse und eine darin angeordnete Medientrennmembran auf. Das Gehäuse weist eine rohrförmige Aufnahme auf zur Aufnahme einer Brusthaube oder eines in eine Brusthaube einsetzbaren flexiblen Brusthaubeneinsatzes, wobei diese Aufnahme einen Aufnahmebereich zur Aufnahme einer Brustwarze einer Mutterbrust definiert und wobei die Medientrennmembran an diese Aufnahme angrenzt oder in sie hineinragt.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: einen Längsschnitt durch eine erfindungsgemässe Brusthaubeneinheit, montiert auf einem Milchsammelbehälter, in einer ersten Ausführungsform;
- Figur 2: einen Längsschnitt durch eine erfindungsgemässe Brusthaubeneinheit, montiert auf einem Milchsammelbehälter, in einer zweiten Ausführungsform;
- Figur 3: die Brusthaubeneinheit gemäss Figur 2 in einer vergrösserten Darstellung;
- Figur 4: eine perspektivische Darstellung der Brusthaubeneinheit gemäss Figur 2 in einer ersten Position;
- Figur 5: die Brusthaubeneinheit gemäss Figur 3 beim Anlegen auf eine Mutterbrust;
- Figur 6: eine perspektivische Darstellung der Brusthaubeneinheit in der Position gemäss Figur 5;
- Figur 7: die Brusthaubeneinheit gemäss Figur 3 bei Auflage auf die Mutterbrust;
- Figur 8: die Brusthaubeneinheit gemäss Figur 3 in einer geneigten Position;
- Figur 9: einen Längsschnitt durch eine hier nicht beanspruchte Brusthaubeneinheit, montiert auf einem Milchsammelbehälter, in einer dritten Ausführungsform und
- Figur 10: einen Längsschnitt durch eine hier nicht beanspruchte Brusthaubeneinheit, montiert auf einem Milchsammelbehälter, in einer vierten Ausführungsform.

Gleiche Teile sind in den Figuren mit denselben Bezugszeichen beschriftet.

### BESCHREIBUNG BEVORZUGTER AUSFÜHRUNGSFORMEN

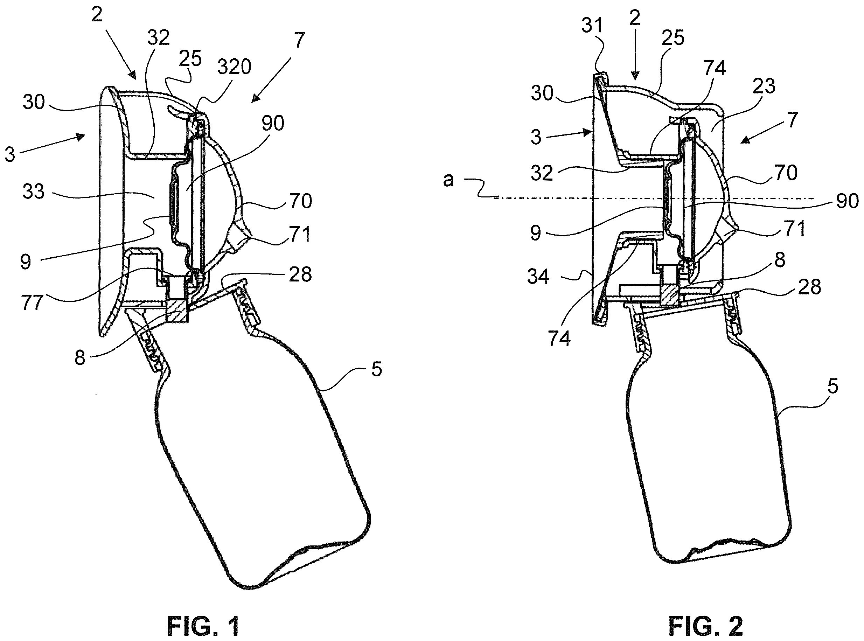

In Figur 1 ist ein erstes Ausführungsbeispiel einer erfindungsgemässen Brusthaubeneinheit dargestellt. Sie weist eine Brusthaube 2 und eine mit ihr verbundene Medientrenneinrichtung 7 auf.

Die Brusthaube 2 weist in dieser Ausführungsform einen formstabilen oder steifen Grundkörper 25, vorzugsweise aus Kunststoff, auf. In diesem Grundkörper 25 ist ein Gehäuse 70 der Medientrenneinrichtung 7 gehalten. Das Gehäuse 70 kann, wie anhand des nachfolgenden Ausführungsbeispiels dargelegt ist, verschiebbar im Grundkörper 25 angeordnet sein. Es kann jedoch auch fest und unverschiebbar mit diesem verbunden sein.

Der Grundkörper 25 weist einen Milchanschlussteil 28 mit einer Milchauslassöffnung auf, welcher zur Verbindung mit einem Milchsammelbehälter 5 dient. Am Gehäuse 70 der Medientrenneinrichtung oder auch am Milchanschlussteil 28 ist ein Ventil 8, vorzugsweise ein Rückschlagventil, angeordnet, um das Totvolumen beim Abpumpen zu begrenzen und ein Rückfliessen der abgepumpten Milch zu verhindern. In diesem Beispiel ist ein Entenschnabelventil dargestellt.

Der Milchanschlussteil 28 und der Milchsammelbehälter 5 können auch eine andere Form aufweisen. Das Milchanschlussteil 28 kann auch Bestandteil der Medientrenneinrichtung 7 sein oder er kann als Deckel des Milchsammelbehälters ausgebildet sein. Als Milchsammelbehälter lassen sich beispielsweise Flaschen oder Beutel einsetzen.

In der Brusthaube 2 ist ein Brusthaubeneinsatz 3 angeordnet. Er ist vorzugsweise aus einem flexiblen Material, insbesondere aus Silikon, gefertigt. Er weist eine Brustauflage 30, hier in Form eines Trichterkörpers, auf, welcher am brustnahen Ende am Grundkörper 25 anliegt oder an ihm lösbar befestigt ist. Die Brustauflage 30 geht vorzugsweise einstückig in einen Stutzen 32 über, welcher in diesem Beispiel am Gehäuse 70 der Medientrenneinrichtung 7 befestigt ist. Diese Art der Befestigung ist insbesondere dann vorteilhaft, wenn, wie nachfolgend erläutert ist, die Medientrenneinrichtung 7 relativ zum Grundkörper 25 der Brusthaube 2 verschiebbar ist. Die Befestigung kann auf verschiedene Arten erfolgen. In diesem Beispiel weist der Stutzen 32 einen brustfernen Flansch 320 auf, welcher in einer entsprechenden Aufnahme des Gehäuses 70 gehalten, insbesondere eingeschnappt, ist.

Die Brustauflage 30 ist vorzugsweise mindestens teilweise elastisch ausgebildet. Sie kann jedoch auch formstabil und sogar steif ausgebildet sein. Der Stutzen 32 ist vorzugsweise formstabiler ausgebildet, beispielsweise indem er zwar aus demselben Material wie die Brustauflage 30 gefertigt ist, aber eine grössere Wandstärke aufweist.

Die Brustauflage 30 dient zur Auflage auf eine Mutterbrust, wobei die Brustwarze der Brust bis in den Stutzen 32 hineinragt. Dadurch bildet der Stutzen 32 mit seinem Hohlraum einen Aufnahmebereich 33 zur Aufnahme der Brustwarze.

Das Gehäuse 70 der Medientrennrichtung 7 ist vorzugsweise steif oder formstabil und insbesondere aus Kunststoff ausgebildet. Am Gehäuse 70 der Medientrenneinrichtung 7 ist ein Vakuumanschluss 71 zur Verbindung mit einer nicht dargestellten Vakuumpumpe vorhanden. Die Verbindung kann direkt oder über eine Saugleitung erfolgen. Die Vakuumpumpe kann manuell oder motorisch betrieben sein. Am Gehäuse 70 ist ferner eine Milchauslassöffnung 77 vorhanden, welche mit dem genannten Ventil 8 verschlossen ist. In weiter unten beschriebenen Ausführungsformen ist diese Milchauslassöffnung in einer Brusthaubeneinsatz-Aufnahme 74 der Medientrenneinrichtung angeordnet. Somit ist ein Milchflussweg gebildet, durch welchen abgepumpte Muttermilch vom Aufnahmebereich 33 durch die Milchauslassöffnung 77, das Ventil 8 und den Milchauslass 28 des Grundkörpers 25 in den Milchsammelbehälter 5 gelangen kann.

Die Medientrenneinrichtung 7 weist eine Medientrennmembran 9 auf, welche im Gehäuse 70 bewegbar gehalten ist. In diesem Beispiel ist sie zwischen einem brustfernen Flansch 320 des Stutzens 32 und dem Gehäuse 70 eingeklemmt. Die Medientrennmembran 9 bildet eine brustnahe Wand und das Gehäuse 70 die brustferne Wand einer Pumpkammer 90. Die Medientrennmembran 9 ist vorzugsweise aus Silikon gefertigt.

Sie dient zur Übertragung eines über den Vakuumanschluss 71 angelegten zyklisch sich verändernden Unterdrucks in den Aufnahmebereich 33 des Brusthaubeneinsatzes 3 bzw. der Brusthaube 2. Wird über den Vakuumanschluss 71 zyklisch ein Unterdruck in die Pumpkammer 90 geleitet, so bewegt sich die Medientrennmembran 9 synchron dazu und überträgt den Unterdruck in einen Hohlraum, gebildet durch den Brusthaubeneinsatz 3 und die Mutterbrust B. Milch wird abgepumpt. Die Medientrennmembran 9 verhindert dabei, dass abgepumpte Milch in die Pumpkammer 90 und somit zur Vakuumquelle gelangen kann. Die Milch fliesst vielmehr durch die der Membran 9 vorgelagerte Milchauslassöffnung 77 in den Milchsammelbehälter 5.

Die Medientrennmembran 9 bewegt sich somit zwischen zwei Positionen hin und her. Die erste Position ist in den Figuren nicht dargestellt. Sie ist brustfern und wird erreicht, wenn ein Unterdruck anliegt. In dieser ersten Position befindet sich die Medientrennmembran 9 im hinteren Bereich der Pumpkammer 90, d.h. bei der hinteren Wandung des Gehäuses 70. Die zweite Position ist in den Figuren dargestellt. Sie ist brustnah.

Die Medientrennmembran 9 ist so angeordnet, dass sie sich mindestens in einer dieser zwei Positionen im Aufnahmebereich 33 befindet. Üblicherweise ragt sie mindestens in der brustnahen zweiten Position in diesen Aufnahmebereich 33 hinein und vermindert so das Totvolumen im genannten Hohlraum.

In den Figuren 2 bis 8 ist ein weiteres Ausführungsbeispiel dargestellt. Gleiche Teile sind mit gleichen Bezugszeichen wie im ersten Beispiel versehen.

In dieser Ausführungsform weist die Brusthaube 2 ebenfalls einen gehäuseförmigen Grundkörper 25 auf. Die Brusthaube 2 dient ebenfalls zur Auflage auf die Mutterbrust B und gleichzeitig zur Verbindung mit dem Milchsammelbehälter 5. Sie weist ebenfalls ein Milchanschlussteil 28 auf, mit welchem sie mit dem Milchsammelbehälter 5 verbunden werden kann. Die Brusthaube 2 ist vorzugsweise steif oder formstabil und insbesondere aus Kunststoff gefertigt. Dasselbe gilt für das Gehäuse 70 der Medientrenneinrichtung, welche im Grundkörper 25 angeordnet ist.

Die Milchauslassöffnung 77, welche mit dem Rückschlagventil 8 versehen ist, führt durch eine Durchführungsöffnung 29 des Grundkörpers 25 in den Milchsammelbehälter 5.

Am Gehäuse 70 der Medientrenneinrichtung 7 ist eine Brusthaubeneinsatz-Aufnahme 74 angeformt, welche einen Aufnahmestutzen für den Stutzen 32 des Einsatzes 3 bildet. Der Stutzen 32 ist in dieser Ausführungsform als freies Ende ausgebildet und weist keinen brustfernen Flansch auf. Der Stutzen 32 des Einsatzes 3 ist bei Gebrauch unverschieblich in dieser Aufnahme 74 gehalten. Er ist beispielsweise in einem Presssitz gehalten und kann zwecks Reinigung oder Ersatz entfernt werden. Er kann jedoch auch nicht zerstörungsfrei lösbar mit der Medientrenneinrichtung 7 verbunden sein. Ein an einem ersten Einsatzende 34 des Einsatzes 3 angeformter Befestigungskragen 31 ist über einen Flansch 22 des Grundkörpers 25 der Brusthaube 2 gestülpt.

Der Einsatz 3 ist vorzugsweise ähnlich ausgebildet wie der Einsatz gemäss dem ersten Beispiel. Vorzugsweise ist der Auflagebereich 30 leicht trichterförmig ausgebildet. Er kann auch annähernd bzw. ganz als plane Ebene ausgebildet sein. Vorzugsweise ist die Länge des Auflagebereichs 30 relativ kurz, d.h. ein Vielfaches kürzer als die gesamte Länge des Einsatzes 3.

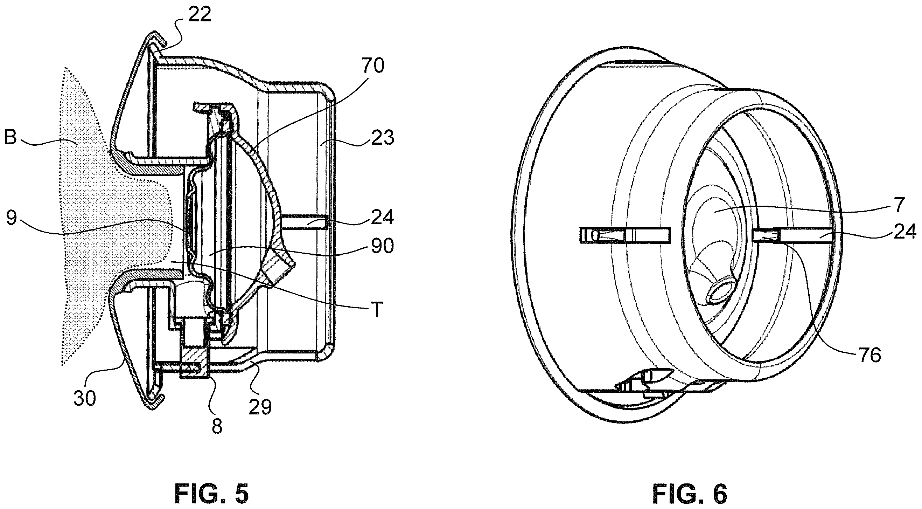

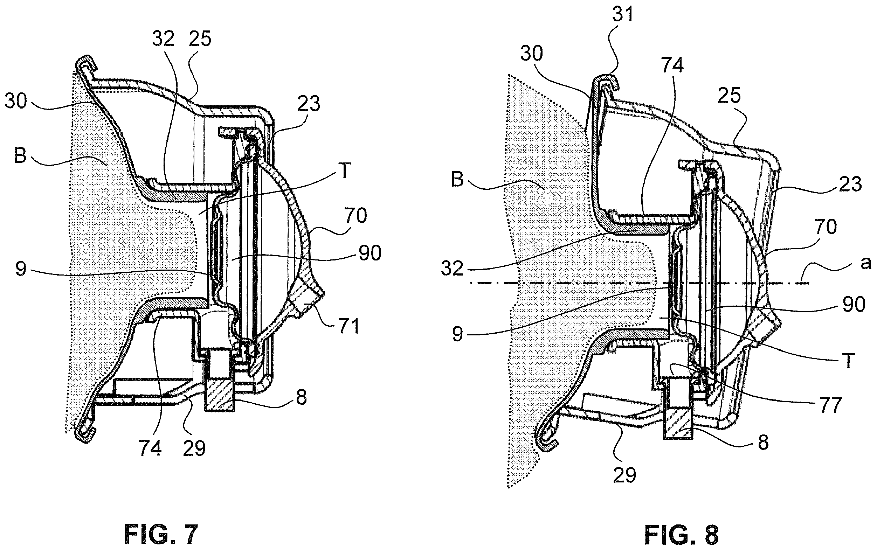

Wie in Figur 5 erkennbar ist, reicht die Medientrennmembran 9 bereits im nicht ausgelenkten Zustand annähernd oder genau bis zum Ende des Stutzens 32 des Einsatzes 3. In Figur 7 ist erkennbar, dass der Stutzen 32 so bemessen ist, dass er bei einer im Normgrössenbereich liegenden Brust und Brustwarze in seiner gesamten Länge von der Brustwarze ausgefüllt ist. Die Medientrennmembran 9 berührt jedoch während des Abpumpens die Brustwarze vorzugsweise nicht, sondern sie bewegen sich synchron in dieselbe Richtung.

Die Medientrenneinrichtung 7 ist vorzugsweise beweglich, insbesondere verschiebbar im Grundkörper 25 der Brusthaube 2 angeordnet. Dies kann auch im Beispiel gemäss Figur 1 der Fall sein.

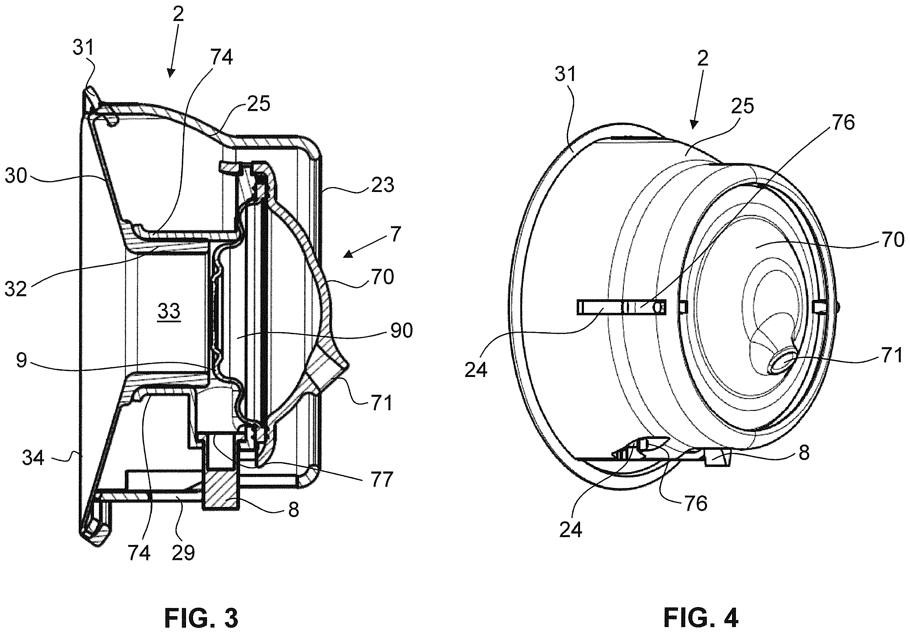

Hierzu weist der Grundkörper 25 Führungsschlitze 24 auf, in welche Führungsnasen 76 des Gehäuses 70 eingreifen. Dies ist beispielsweise in Figur 4 erkennbar. Die Medientrenneinrichtung 7 lässt sich entlang dieser Schlitze 24 verschieben. Die Schlitze 24 können geradlinig oder gekrümmt verlaufen. In diesem Beispiel sind sie geradlinig. Das Ventil 8 und die Milchauslassöffnung 77 führen in allen Positionen der Medientrenneinrichtung 7 relativ zum Grundkörper 25 in den Milchsammelbehälter 5, da die Durchführöffnung 29 grösser ausgebildet ist als das Ventil 8 und die Milchauslassöffnung 77. Vorzugsweise ist die Durchführungsöffnung 29 als Langloch ausgebildet.

Das hintere, der Brust abgewandte Ende des Grundkörpers 25 ist offen ausgebildet. Die Medientrenneinrichtung 7 lässt sich manuell über diese rückseitige Öffnung 23 bewegen, hier entlang der Schlitze 24 verschieben.

In den Figuren 2 bis 4 ist die Brusthaubeneinheit im unbenützten bzw. aktuell nicht benützten Zustand dargestellt. Die Medientrenneinrichtung 7 ragt durch die rückseitige Öffnung 23 heraus oder befindet sich zumindest in einem hinteren Bereich. Die Brustauflage 30 des Einsatzes 3 ist entspannt bzw. nicht ausgelenkt und in diesem Beispiel leicht trichterförmig.

Um die Brusthaubeneinheit zu verwenden, wird die Medientrenneinrichtung 7 von Hand nach vorne gestossen, wie dies in den Figuren 5 und 6 dargestellt ist. Die Brustauflage 30 des Einsatzes 3 wird gedehnt, je nach Bedarf nach vorne zur Mutterbrust B hin gestülpt. Die Brustwarze kann optimal in der Aufnahmeöffnung 33 aufgenommen werden. Wird nun der manuelle Druck auf das Gehäuse 70 gelöst, der Grundkörper 25 jedoch nach wie vor von Hand, in einem Büstenhalter oder einer anderen Haltevorrichtung an die Brust gehalten, so schiebt sich das Gehäuse 70 entlang der Schlitze 24 nach hinten und der Einsatz 3 liegt dichtend, jedoch ohne übermässigen Druck an der Brust B an. Dies ist in Figur 7 dargestellt. Das Abpumpen kann beginnen.

Das zweite Einsatzende des Brusthaubeneinsatzes 3, d.h. der Stutzen 32, ist somit relativ zur Brusthaube 2 verschiebbar, wobei die Verschiebung gemeinsam mit der Medientrenneinrichtung 7 erfolgt.

Je nach Form der Führungsschlitze 24 ist es auch möglich, die Medientrenneinrichtung 7 relativ zur Brusthaube 2 zu neigen. Dies ist in Figur 8 dargestellt. Vorzugsweise lässt sie sich in verschiedene Richtungen neigen.

In den zwei oben genannten Ausführungsformen ist die Medientrennmembran 9 annähernd senkrecht zum Anschlussteil 28, d.h. annähernd senkrecht zur Längsrichtung a der Brusthaube 2 und des Brusthaubeneinsatzes 3, angeordnet.

In den zwei nachfolgend beschriebenen Ausführungsbeispielen ist die Medientrennmembran hingegen annähernd horizontal, d.h. annähernd parallel zum Milchanschlussteil bzw. im Milchflussweg angeordnet.

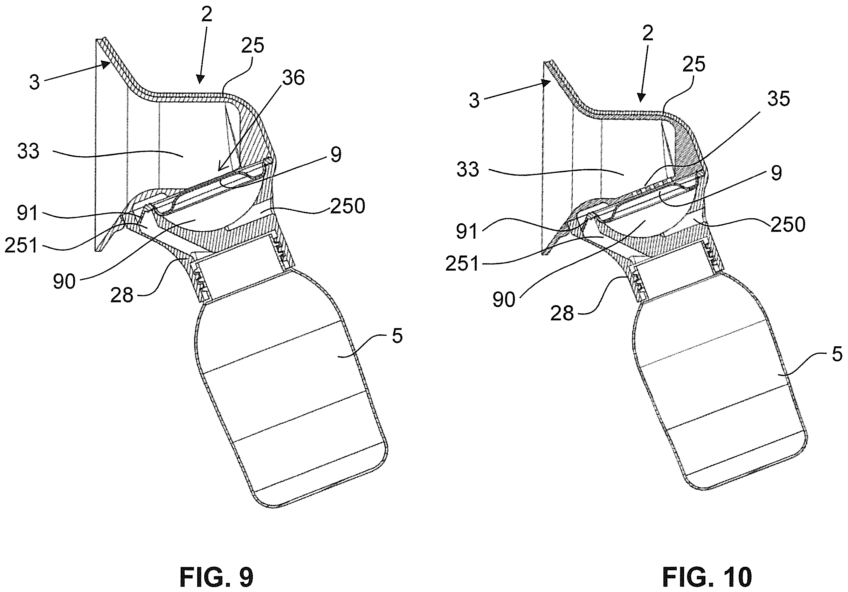

In Figur 9 ist wiederum eine Brusthaube 2 mit einer Medientrennmembran 9, montiert auf einem Milchsammelbehälter 5, dargestellt.

Die Brusthaube 2 weist wiederum den formstabilen, vorzugsweise steifen Grundkörper 25 mit einem Milchanschlussteil 28 zur Befestigung auf dem Milchsammelbehälter 5 auf. Im Grundkörper 5 ist wiederum ein Brusthaubeneinsatz 3 angeordnet, welcher vorzugsweise flexibel ausgebildet ist. Er kann jedoch auch formstabil, insbesondere steif ausgebildet sein. Ebenso kann er einstückig mit der Brusthaube 2 ausgebildet sein. Der Einsatz 3 definiert an seinem brustfernen, geschlossenen Ende 26 den Aufnahmebereich 33. Eine untere Wand des Einsatzes 3 bzw. der Brusthaube 2 ist als Durchlassöffnung 36 ausgebildet. Die Wand ist dabei vorzugsweise partial als Durchlassöffnung ausgebildet. Unterhalb dieser Öffnung ist die Medientrennmembran 9 angeordnet. Sie ist vorzugsweise zwischen Brusthaube 2 und Einsatzelement 3 eingeklemmt gehalten. Die Medientrennmembran 9 verschliesst die Öffnung 36, wobei sie je nach angelegtem Unterdruck diese frei gibt und somit einen Milchflussweg zwischen Aufnahmebereich 33 und Milchsammelbehälter 5 öffnet.

Unterhalb der Durchlassöffnung 36 und der Medientrennmembran 9 befindet sich die Pumpkammer 90, von welcher ein Vakuumkanal 250 nach aussen führt. Die übrige Wand der Pumpkammer 90 ist vom Grundkörper 25, bzw. vom Milchanschlussteil 28 gebildet. Der Vakuumkanal 250 verläuft im Grundkörper 25 bzw. im Milchanschlussteil 28. Somit bildet die Brusthaube 2 auch die Medientrenneinrichtung.

Der Milchflussweg oder Milchkanal 251 führt von der Durchlassöffnung 36 zum Milchsammelbehälter 5. Er ist aufgrund der Medientrennmembran 9 seitlich umgelenkt und verläuft, vorzugsweise im brustnahen Bereich, seitlich zur Pumpkammer 90. Dabei verläuft er vorzugsweise durch den in Gebrauchslage tiefsten Punkt der Medientrennmembran, damit keine Milch auf der Membran liegen bleibt. Dieser Milchkanal 251 ist vorzugsweise durch ein Rückschlagventil 91 verschliessbar. Das Rückschlagventil 91 ist vorzugweise einstückig mit der Medientrennmembran 9 ausgebildet.

Die Ausführungsform gemäss Figur 10 entspricht im Wesentlichen derjenigen gemäss Figur 9. Hier ist die untere Wand des Einsatzes 3 jedoch bis auf einige Durchbrüche 35 geschlossen ausgebildet. Dadurch ist eine obere Begrenzung für die Medientrennmembran 9 gegeben und die Brustwarze kann diese Membran 9 nicht kontaktieren.

Die erfindungsgemässe Brusthaubeneinheit lässt sich trotz integrierter Medientrennung äusserst kompakt herstellen und minimiert das Totvolumen.

**BEZUGSZEICHENLISTE**

| | | | |
|---|---|---|---|
| 2 | Brusthaube | | |
| 22 | Flansch | 5 | Milchsammelbehälter |
| 23 | rückseitige Öffnung | | |
| 24 | Führungsschlitz | 7 | Medientrenneinrichtung |
| 25 | Grundkörper | 70 | Gehäuse |
| 250 | Vakuumkanal | 71 | Vakuumanschluss |
| 251 | Milchkanal | 74 | Brusthaubeneinsatz-Aufnahme |
| 26 | geschlossenes Ende | | |
| 28 | Milchanschlussteil | 76 | Führungsnase |
| 29 | Durchführungsöffnung | 77 | Milchauslassöffnung |
| | | | |
| 3 | Brusthaubeneinsatz | 8 | Ventil |
| 30 | Brustauflage | | |
| 31 | Befestigungskragen | 9 | Medientrennmembran |
| 32 | Stutzen | 90 | Pumpkammer |
| 320 | Flansch | 91 | Rückschlagventil |
| 33 | Aufnahmeöffnung | | |
| 34 | erstes Einsatzende | B | Brust |
| 35 | Durchbrüche | T | Totvolumen |
| 36 | Durchlassöffnung | a | Längsrichtung |

## Patentansprüche

1. Brusthaubeneinheit zur Verwendung mit einer Brustpumpe zum Abpumpen von menschlicher Muttermilch, wobei die Brusthaubeneinheit eine Brusthaube (2) zur Aufnahme einer Mutterbrust (B) und eine Medientrenneinrichtung (7, 9) zur Übertragung eines Vakuums in das Innere der Brusthaube (2) aufweist, wobei die Brusthaubeneinheit einen Aufnahmebereich (33) zur Aufnahme einer Brustwarze der Mutterbrust (B) aufweist und wobei die Medientrenneinrichtung (7, 9) eine Medientrennmembran (9) aufweist, welche zwecks Übertragung des Vakuums zwischen zwei Positionen hin und her bewegbar ist, wobei sich die Medientrennmembran (9) mindestens in einer der zwei genannten Positionen im Aufnahmebereich (33) befindet, wobei ein rohrförmiger Stutzen (32) vorhanden ist, welcher den Aufnahmebereich (33) bildet und welcher ein brustfernes Ende aufweist, wobei die Medientrennmembran (9) unmittelbar an diesem Ende angeordnet ist, **dadurch gekennzeichnet, dass** die Medientrenneinrichtung (7, 9) ein formstabiles Gehäuse (7) mit einem Vakuumanschluss (71) zur Verbindung mit einer Vakuumpumpe -aufweist, wobei die Medientrennmembran (9) bewegbar im Gehäuse (7) gehalten ist, und dass die Medientrenneinrichtung (7, 9) als Ganzes relativ zur Brusthaube (2) in dieser bewegbar angeordnet ist.

2. Brusthaubeneinheit nach Anspruch 1, wobei die Brusthaubeneinheit einen Anschluss (28) zur Verbindung mit einem Milchsammelbehälter (5) aufweist und wobei die Medientrennmembran (9) annähernd senkrecht zu diesem Anschluss (28) angeordnet ist.

3. Brusthaubeneinheit nach Anspruch 1, wobei ein Milchflussweg vorhanden ist, welcher vom Aufnahmebereich (33) zu einem Anschluss (28) zur Verbindung mit einem Milchsammelbehälter (5) führt, und wobei die Medientrennmembran (9) in diesem Milchflussweg angeordnet ist.

4. Brusthaubeneinheit nach Anspruch 3, wobei die Medientrennmembran (9) nach Massgabe eines zyklisch angelegten Unterdrucks den Milchflussweg unterbricht und wieder freigibt.

5. Brusthaubeneinheit nach einem der Ansprüche 1 oder 3 bis 4, wobei die Brusthaubeneinheit einen Anschluss (28) zur Verbindung mit einem Milchsammelbehälter (5) aufweist und wobei die Medientrennmembran (9) annähernd waagrecht zu diesem Anschluss (28) angeordnet ist.

6. Brusthaubeneinheit nach einem der Ansprüche 1 bis 5, wobei in einem Milchflussweg ein Rückschlagventil (91) angeordnet ist, und wobei das Rückschlagventil (91) einstückig mit der Medientrennmembran (9) ausgebildet ist.

7. Brusthaubeneinheit nach Anspruch 1, wobei sie ferner einen Brusthaubeneinsatz (3) aufweist, welcher in der Brusthaube (2) befestigbar ist und wobei die Medientrenneinrichtung (7, 9) mittels dieses Brusthaubeneinsatzes (3) in der Brusthaube (2) bewegbar aufgehängt ist.

8. Brusthaubeneinheit nach einem der Ansprüche 1 bis 7, wobei die Brusthaube (2) sich in einer Längsrichtung (a) der Brusthaube (2) erstreckende Führungsschlitze (24) aufweist, entlang welcher die Medientrenneinrichtung (7, 9) als Ganzes bewegbar ist.

9. Brusthaubeneinheit nach einem der Ansprüche 1 bis 8, wobei die Brusthaube (2) an einem der Mutterbrust (B) abgewandten Ende eine rückseitige Öffnung (23) aufweist, wodurch die Medientrenneinrichtung (7, 9) durch Zugang über diese rückseitige Öffnung (23) zur Mutterbrust (B) hin manuell verschiebbar ist.

10. Brusthaubeneinheit nach einem der Ansprüche 1 bis 9, wobei die Brusthaube (2) formstabil ausgebildet ist und wobei die Brusthaubeneinheit ferner einen flexiblen Brusthaubeneinsatz (3) zur Einlage in die Brusthaube (2) aufweist, wobei die Brusthaube (2) ein erstes offenes Brusthaubenende zur Anlage an eine Mutterbrust (B) aufweist, wobei der Brusthaubeneinsatz (3) ein erstes und ein zweites Einsatzende (34, 32) aufweist, welche eine Längsrichtung (a) definieren, wobei das erste offene Brusthaubenende einen umlaufenden Rand (22) aufweist, an welchem der Brusthaubeneinsatz (3) mit dem ersten Einsatzende (31) befestigbar oder befestigt ist, wobei sich der Brusthaubeneinsatz (3) im montierten Zustand von diesem ersten Einsatzende (31) im Innern der Brusthaube (2) zum zweiten Einsatzende (32) erstreckt, wobei der Brusthaubeneinsatz (3) zwischen dem ersten und dem zweiten Einsatzende (31, 32) im Wesentlichen beabstandet zur Brusthaube (2) verläuft, und wobei das zweite Einsatzende (32) im montierten Zustand des Brusthaubeneinsatzes (3) in Längsrichtung (a) relativ zur Brusthaube (2) verschiebbar ist.

## Claims

1. Breastshield unit for use with a breastpump for expressing human breastmilk, wherein the breastshield unit has a breastshield (2) for receiving a mother's breast (B) and a media separation device (7, 9) for transferring a vacuum into the interior of the breastshield (2), wherein the breastshield unit has a receiving area (33) for receiving a nipple of the mother's breast (B), and wherein the media separation device (7, 9) has a media separation membrane (9) which, for the purpose of transferring the vacuum, is movable to and fro between two positions, wherein the media separation membrane (9) is located in the receiving area (33) at least in one of said two positions, wherein a tubular stub (32) is present which forms the receiving area (33) and which has an end directed away from the breast, wherein the media separation membrane (9) is arranged directly on this end, **characterized in that** the media separation device (7, 9) has a dimensionally stable housing (7) with a vacuum connector (71) for connection to a vacuum pump, wherein the media separation membrane (9) is held movably in the housing (7) and that the media separation device (7, 9) as a whole is arranged to be movable relative to and inside the breastshield (2).

2. Breastshield unit according to Claim 1, wherein the breastshield unit has an attachment (28) for connection to a milk collection container (5), and wherein the media separation membrane (9) is arranged approximately perpendicularly with respect to this attachment (28).

3. Breastshield unit according to Claim 1, wherein a milk flow path is present which leads from the receiving area (33) to an attachment (28) for connection to a milk collection container (5), and wherein the media separation membrane (9) is arranged in this milk flow path.

4. Breastshield unit according to Claim 3, wherein the media separation membrane (9) interrupts and re-opens the milk flow path according to a cyclically applied underpressure.

5. Breastshield unit according to one of Claims 1 or 3 to 4, wherein the breastshield unit has an attachment (28) for connection to a milk collection container (5), and wherein the media separation membrane (9) is arranged approximately horizontally with respect to this attachment (28).

6. Breastshield unit according to one of Claims 1 to 5, wherein a check valve (91) is arranged in a milk flow path, and wherein the check valve (91) is formed in one piece with the media separation membrane (9).

7. Breastshield unit according to Claim 1, wherein it also has a breastshield insert (3) which can be secured in the breastshield (2), and wherein the media separation device (7, 9) is suspended movably by means of this breastshield insert (3) in the breastshield (2).

8. Breastshield unit according to either of Claims 1 to 7, wherein the breastshield (2) has guide slits (24) which extend in a longitudinal direction (a) of the breastshield (2) and along which the media separation device (7, 9) as a whole is movable.

9. Breastshield unit according to one of Claims 1 to 8, wherein the breastshield (2) has a rear opening (23) at an end directed away from the mother's breast (B), as a result of which the media separation device (7, 9) is manually movable towards the mother's breast (B) by access through this rear opening (23).

10. Breastshield unit according to one of Claims 1 to 9, wherein the breastshield (2) is dimensionally stable, and wherein the breastshield unit also has a flexible breastshield insert (3) for insertion into the breastshield (2), wherein the breastshield (2) has a first, open breastshield end for placing onto a mother's breast (B), wherein the breastshield insert (3) has a first and a second insert end (34, 32) which define a longitudinal direction (a), wherein the first, open breastshield end has a peripheral edge (22) on which the breastshield insert (3) can be secured or is secured with the first insert end (31), wherein the breastshield insert (3), in the assembled state, extends from this first insert end (31) in the interior of the breastshield (2) to the second insert end (32), wherein the breastshield insert (3), between the first and second insert ends (31, 32), extends substantially spaced apart from the breastshield (2), and wherein the second insert end (32), in the assembled state of the breastshield insert (3), is movable in the longitudinal direction (a) relative to the breastshield (2).

## Revendications

1. Unité téterelle à utiliser avec un tire-lait pour soutirer du lait maternel humain, dans laquelle l'unité téterelle présente une téterelle (2) destinée à recevoir un sein maternel (B) et un dispositif de séparation de milieux (7, 9) pour la transmission d'un vide à l'intérieur de la téterelle (2), dans laquelle l'unité téterelle présente une zone de réception (33) pour la réception d'un mamelon du sein maternel (B) et dans laquelle le dispositif de séparation de milieux (7, 9) présente une membrane de séparation de milieux (9), qui peut se déplacer en va-et-vient entre deux positions pour la transmission du vide, dans laquelle la membrane de séparation de milieux (9) se trouve au moins dans une des deux positions précitées dans la zone de réception (33), dans laquelle il se trouve un embout tubulaire (32) qui forme la zone de réception (33) et qui présente une extrémité éloignée du sein, dans laquelle la membrane de séparation de milieux (9) est disposée directement à cette extrémité, **caractérisée en ce que** le dispositif de séparation de milieux (7, 9) présente un boîtier de forme stable (7) avec un raccord de vide (71) pour le raccordement à une pompe à vide, dans laquelle la membrane de séparation de milieux (9) est maintenue de façon mobile dans le boîtier (7) et **en ce que** le dispositif de séparation de milieux (7, 9) est disposé en bloc dans la téterelle (2) de façon mobile par rapport à celle-ci.

2. Unité téterelle selon la revendication 1, dans laquelle l'unité téterelle présente un raccord (28) pour le raccordement à un récipient de collecte du lait (5) et dans laquelle la membrane de séparation de milieux (9) est disposée à peu près perpendiculairement à ce raccord (28).

3. Unité téterelle selon la revendication 1, dans laquelle il se trouve un chemin d'écoulement du lait, qui conduit de la zone de réception (33) à un raccord (28) pour le raccordement à un récipient de collecte du lait (5), et dans laquelle la membrane de séparation de milieux (9) est disposée dans ce chemin d'écoulement du lait.

4. Unité téterelle selon la revendication 3, dans laquelle la membrane de séparation de milieux (9) interrompt et rétablit le chemin d'écoulement du lait selon une dépression appliquée de façon cyclique.

5. Unité téterelle selon l'une quelconque des revendications 1 ou 3 à 4, dans laquelle l'unité téterelle présente un raccord (28) pour le raccordement à un récipient de collecte du lait (5) et dans laquelle la membrane de séparation de milieux (9) est disposée à peu près horizontalement à ce raccord (28).

6. Unité téterelle selon l'une quelconque des revendications 1 à 5, dans laquelle un clapet antiretour (91) est disposé dans le chemin d'écoulement du lait, et dans laquelle le clapet antiretour (91) est formé d'une seule pièce avec la membrane de séparation de milieux (9).

7. Unité téterelle selon la revendication 1, dans laquelle elle présente en outre un insert de téterelle (3), qui peut être fixé dans la téterelle (2) et dans laquelle le dispositif de séparation de milieux (7, 9) est suspendu de façon mobile dans la téterelle (2) au moyen de cet insert de téterelle (3).

8. Unité téterelle selon l'une quelconque des revendications 1 à 7, dans laquelle la téterelle (2) présente des fentes de guidage (24) s'étendant dans une direction longitudinale (a) de la téterelle (2), le long desquelles le dispositif de séparation de milieux (7, 9) est déplaçable en bloc.

9. Unité téterelle selon l'une quelconque des revendications 1 à 8, dans laquelle la téterelle (2) présente à une extrémité éloignée du sein maternel (B) une ouverture arrière (23), à travers laquelle le dispositif de séparation de milieux (7, 9) peut être déplacé manuellement vers le sein maternel (B) par accès via cette ouverture arrière (23).

10. Unité téterelle selon l'une quelconque des revendications 1 à 9, dans laquelle la téterelle (2) est réalisée sous forme stable et dans laquelle l'unité téterelle présente en outre un insert de téterelle flexible (3) à placer dans la téterelle (2), dans laquelle la téterelle (2) présente une première extrémité de téterelle ouverte pour l'application sur un sein maternel (B), dans laquelle l'insert de téterelle (3) présente une première et une deuxième extrémités d'insert (34, 32), qui définissent une direction longitudinale (a), dans laquelle la première extrémité de téterelle ouverte présente un bord périphérique (22), sur lequel l'insert de téterelle (3) peut être ou est fixé avec la première extrémité d'insert (31), dans laquelle l'insert de téterelle (3) s'étend dans l'état monté depuis cette première extrémité d'insert (31) à l'intérieur de la téterelle (2) jusqu'à la deuxième extrémité d'insert (32), dans laquelle l'insert de téterelle (3) s'étend entre la première et la deuxième extrémités d'insert (31, 32) essentiellement à distance de la téterelle (2), et dans laquelle la deuxième extrémité d'insert (32) peut être déplacée dans la direction longitudinale (a) par rapport à la téterelle (2) dans l'état monté de l'insert de téterelle (3).
